# EUROPEAN PATENT APPLICATION

(11) **EP 3 750 573 A1**
(43) Date of publication of application: **16.12.2020**
(21) Application number: 19305760.1
(22) Date of filing: 14.06.2019
(51) Int. Cl.: A61M 5/145, A61M 5/142, A61M 5/20

(54) **INFUSION DEVICE HAVING A BRAKE DEVICE**

(71) Applicant: Fresenius Vial SAS, 38590 Brézins (FR)
(72) Inventor: TERRIER, David, 38590 Sillans (FR); WOLFF, Rémy, 38210 Morette (FR)
(74) Representative: Fresenius Kabi Deutschland GmbH

(57) **Abstract**

An infusion device (1) for administering a medical fluid to a patient comprises: a receptacle (11) for receiving a syringe (2), a pusher device (12) movable in a pushing direction (P) for acting onto a piston (21) of a syringe (2) received in the receptacle (11), a fastening element (122) arranged on the pusher device (12) and movable in between a non-actuated position and a fully actuated position with respect to the pusher device (12), a brake device (16) for braking a movement of the pusher device (12), and a control device (17) for controlling operation of the brake device (16). Further, the infusion device (1) comprises a fastening element sensing device (173) for sensing a position state of the fastening element (122) with respect to the pusher device (12), wherein the control device (17) is configured to control the brake device (16), based on an output of the fastening element sensing device (173), to assume a braking state for braking a movement of the pusher device (12) or a non-braking state for allowing a movement of the pusher device (12).

## Description

The invention relates to an infusion device for administering a medical fluid to a patient according to the preamble of claim 1.

An infusion device of this kind comprises a receptacle for receiving a syringe, and a pusher device movable in a pushing direction for acting onto a piston of a syringe received in the receptacle. A fastening element is arranged on the pusher device and is movable in between a non-actuated position and a fully actuated position with respect to the pusher device. A brake device serves to brake a movement of the pusher device. Operation of the brake device herein is controlled by a control device.

Within an infusion device in the shape of a syringe pump, as it for example is known from US 2012/015170 A1 and WO2018046313A1, a pusher device serves to act onto a piston of a syringe in order to push, during operation, the piston of the syringe into a syringe tube in order to deliver a medical fluid from the syringe towards a patient in the context of an ongoing infusion operation. The piston herein, during installation of the syringe, is brought into operative connection with the pusher device, such that during operation of the infusion device the pusher device may transfer a force to the piston.

In order to fasten the piston to the pusher device, in particular to also be able to cause a movement of the piston opposite to the pushing direction, a fastening element, for example in the shape of a lever (also denoted as anti-siphon arm), is used which engages with the piston of the syringe in order to lock the piston with respect to the pusher device and to hold the piston in abutment with the pusher device.

For installing a syringe on the receptacle of the infusion device, the fastening element can be actuated and hence moved with respect to the pusher device such that the pusher device may be brought into abutment with the piston of the syringe. Once the pusher device contacts the piston, an actuation element for actuating the fastening element may be released such that the fastening element may engage with the piston and may cause a locking of the piston with respect to the pusher device.

Generally, once it is detected that the pusher device during installation of a syringe on the infusion device has come into contact with the piston of the syringe, the brake device is actuated such that a further movement of the pusher device is blocked. In this way an undesired bolus during installation of the syringe is avoided, in that the pusher device can be brought into abutment with the piston of the syringe, but cannot be moved further once a contact between the pusher device and the piston of the syringe has been established.

However, a scenario may arise in which a user during installation of a syringe does not fully actuate the fastening element to move it towards its fully actuated position. This may lead to a situation in which a user approaches the piston of the syringe with the pusher device, the piston however not coming into contact with a pusher surface of the pusher device, but with the fastening element. In this case, potentially, a contact between the pusher device and the piston is not detected, such that the brake device may not be actuated and hence a bolus may potentially be administered to a patient upon movement of the pusher device with the fastening element contacting the piston of the syringe.

As in particular in an emergency situation it cannot fully be ruled out that a user may not correctly handle an actuation element for moving the fastening element and hence may not actuate the fastening element correctly during an installation procedure, there is a desire to avoid a situation like this.

It is an object of the instant invention to provide an infusion device and a method for controlling operation of an infusion device which allow in particular an increased safety and improved handling during e.g. an installation of a syringe on the infusion device.

This object is achieved by means of an infusion device comprising the features of claim 1.

Accordingly, the infusion device comprises a fastening element sensing device for sensing a position state of the fastening element with respect to the pusher device, wherein the control device is configured to control the brake device, based on an output of the fastening element sensing device, to assume a braking state for braking a movement of the pusher device or a non-braking state for allowing a movement of the pusher device.

The fastening element sensing device serves to detect a position state of the fastening element, in particular during an installation of a syringe on the receptacle of the infusion device. The fastening element sensing device herein may detect one or multiple discrete positions of the fastening element. For example, the fastening element sensing device may detect whether the non-actuated position or the fully actuated position has been reached, or whether the fastening element is in an intermediate position in between the non-actuated position and the fully actuated position. Alternatively, the fastening element sensing device may detect the position of the fastening element continuously, such that the fastening element sensing device outputs a measurement value indicating the actual position of the fastening element.

The non-actuated position herein may relate to a position in which the fastening element has not been actuated and no syringe is placed on the infusion device, such that the fastening element assumes a default rest position. The fastening element may for example be spring-loaded towards this non-actuated position such that the fastening element automatically assumes the non-actuated position if an actuation device for actuating the fastening element is not actuated.

The fully actuated position in turn may correspond to a position of the fastening element which the fastening element assumes when it is fully raised from the non-actuated position. The fully actuated position in particular corresponds to that position of the fastening element in which the pusher device may be brought into engagement with the piston of a syringe received in the receptacle of the infusion device. In the fully actuated position the fastening element can be moved past a piston head of a piston of a syringe received in the receptacle of the infusion device, such that the fastening element may engage with the piston upon releasing an actuation device for actuating the fastening element.

Because the brake device is controlled by taking into account an output of the fastening element sensing device, it may be detected whether the fastening element has been fully actuated and hence the pusher device can be brought correctly into engagement with the piston of a syringe received in the receptacle of the infusion device. Based on a reading of the fastening element sensing device it can in particular be detected if the fastening element has not been fully actuated and hence for example assumes an intermediate position between the non-actuated position and the fully actuated position. In this case the brake device may be actuated for bringing the brake device into the braking state such that a movement of the pusher device is prevented and the pusher device may not be moved to approach the piston of a syringe received in the receptacle of the infusion device.

The handling of the infusion device in particular for installing a syringe on the infusion device hence can be improved, in that a user intuitively is forced to always fully activate the fastening element (also denoted as anti-siphon arm) for bringing the pusher device into operative connection with the piston of a syringe. If the fastening element has not been fully actuated and a user nevertheless tries to move the pusher device towards the piston of a syringe, movement may be blocked in that the brake device may be actuated.

Alternatively or in addition, a braking of the pusher device in dependence of a position of the fastening element may also be caused for example when uninstalling a syringe. For example, when a syringe shall be uninstalled, but the fastening element is not fully actuated, the fastening element may still be connected to the piston of the syringe, such that a movement of the pusher device for example opposite to the pushing direction would carry the piston along. This can be prevented by transferring the brake device to its braking state and hence braking a movement of the pusher device, such that also during an uninstallation procedure a user is intuitively forced to fully actuate the fastening element to move the fastening element to the fully actuated position.

In one embodiment, the control device is configured to control the brake device based on an output of the fastening element sensing device and an output of at least one further sensing device. For example, in one embodiment, the control device may be configured to control the brake device to assume the braking state if an output of the fastening element sensing device indicates that the fastening element is in an intermediate position in between the non-actuated position and the fully actuated position and if in addition an output of at least one further sensing device indicates that the pusher device is not in operative connection with a piston of a syringe. Hence, the brake device is actuated for example during an installation procedure not only based on an output of the fastening element sensing device, but other sensor readings of other sensor devices are also taken into account. For example, if the infusion device comprises a driving assembly having clutch elements for releasably connecting the driving assembly to a drive device, the brake device is in one embodiment switched to its braking state during an installation of a syringe only if the fastening element is not fully actuated and, in addition, the driving assembly is in an unclutched state, hence allowing for a free movement of the pusher device. By taking into account further sensor readings it can be differentiated between an installation procedure (during which a braking shall be caused) and a normal use situation during normal operation of the infusion device in the context of performing an infusion operation.

In one embodiment, the infusion device comprises a presence sensing device for sensing a presence of a piston of a syringe on the pusher device, wherein the control device is configured to control the brake device to assume the braking state or the non-braking state based in addition on an output of the presence sensing device. When a piston is regularly connected to the pusher device, it may for example abut with a shim element placed on the pusher device, such abutment being detectable by means of a presence sensing device for example in the shape of a touch sensor or an optical sensor such as a photo cell. The presence sensing device hence serves to indicate whether, for example during an installation procedure, a contact between the pusher device and the piston of a syringe has been established. The reading of the presence sensing device may be used for controlling the brake device during an installation procedure, in that for example a braking is only initiated if no presence of a piston on the pusher device is detected, a reading of the presence sensing device hence indicating that the pusher device is not in contact with a piston of a syringe. By means of the presence sensing device and by taking into account a reading of the presence sensing device for a decision whether to initiate a braking action of the brake device during installation of a syringe it is possible to cause a braking only if the pusher device has not yet been brought into operative connection with a piston. If an operative connection between the pusher device and the piston has been established, no braking action during the installation procedure in dependence of a reading of the fastening element sensing device may be required.

In one embodiment, the infusion device comprises a driving rod, an electric drive device for driving the driving rod, and a driving assembly comprising at least one clutch element. The at least one clutch element serves to connect, in a clutched state, the driving rod to the pusher device for driving the pusher device by the electric drive device.

The driving assembly herein can be actuated to assume an unclutched state in which the operative connection between the driving rod and the pusher device is released.

The driving assembly is releasable in order to allow for the installation of the syringe on the infusion device. By transferring the driving assembly from the clutched state into the unclutched state, the operative connection between the driving rod and the pusher device is released, such that the pusher device can be moved independently of the driving rod and hence independently of the electric drive device. This allows to manually move the pusher device into a position in which the syringe can be placed in the receptacle of the infusion device. Upon installation of the syringe, the pusher device is approached towards the piston of the syringe and, for example by releasing an actuation lever, the driving assembly is transferred back to its clutched state such that the operative connection between the driving rod and the pusher device is re-established.

Also, if a user wishes to deliver a manual bolus, the user may unclutch the driving assembly, may administer the bolus and may release an actuation lever to cause clutching of the driving rod to the pusher device, such that the regular infusion procedure may resume.

In one embodiment, the driving assembly comprises a frame member operatively connected to the pusher device and the at least one clutch element is configured to releasably engage with the driving rod. The at least one clutch element may for example comprise a screw thread engaging with a corresponding screw thread of the driving rod, such that a rotational movement of the driving rod is transferred into a longitudinal movement of the frame member along the driving rod, the connection of the frame member to the pusher device causing the pusher device to be moved such that the piston of the syringe is driven by the pusher device. By releasing the at least one clutch element from the driving rod, an independent movement of the pusher device with respect to the driving rod is possible, in particular for installing a syringe on the infusion device.

The at least one clutch element is, in one embodiment, spring elastically tensioned towards the clutched state. The spring elastically tensioning, effected for example by means of a pressure spring, causes the at least one clutch element to move towards the clutched state as soon as an actuation device, for example in the shape of a pivotable lever for example placed on the pusher device, is released. The tensioning of the at least one clutch element causes the driving assembly to regularly assume the clutched state, such that only upon actuation of the actuation device the driving assembly is released and an independent movement of the pusher device from the driving rod becomes possible.

In one embodiment, the driving assembly comprises two clutch elements, each clutch elements being movably arranged on the frame member, wherein the two clutch elements are movable (in particular pivotable) in opposite directions for transferring the driving assembly into the unclutched state.

In one embodiment, the infusion device comprises a clutch sensing device for sensing a state of the driving assembly. In particular, the clutch sensing device may be configured to sense whether the driving assembly is in the clutched state, in which the at least one clutch element is in operative connection with the driving rod, or in the unclutched state, in which the at least one clutch element is released from the driving rod and the driving assembly and hence is freely movable with respect to the driving rod.

An output of the clutch sensing device may in particular be used by the control device for controlling the brake device to assume the braking state in case of an actuation of the fastening element. In particular, when it is detected that the driving assembly is in the unclutched state and the fastening element is in an intermediate position in between the non-actuated position and the fully actuated position, the control device may transfer the brake device into the braking state such that a braking action for braking the pusher device is initiated and a movement of the pusher device hence is prevented. Hence, when the driving assembly is unclutched a movement of the pusher device for example for installing a syringe on the infusion device is possible only if the fastening element has been fully actuated and hence is brought into a position in which the pusher device may be brought into operative connection with the piston of the syringe.

In one embodiment, the infusion device comprises an actuation device for actuating at least one of the fastening element and the driving assembly. The actuation device may have the shape of a lever and may be slidably or pivotably arranged on the pusher device such that the actuation device can be actuated on the pusher device. The actuation device, in one embodiment, serves to simultaneously move the fastening element and the at least one clutch element of the driving assembly, such that by means of the actuation device the fastening element can be moved towards the fully actuated position and, at the same time, the at least one clutching element of the driving assembly may be moved such that the driving assembly assumes the unclutched state.

The infusion device, in one embodiment, may comprise an actuation sensing device for sensing a state of the actuation device. The control device may be configured to control the brake device to assume the braking state or the non-braking state based in addition on an output of the actuation sensing device. The actuation sensing device may for example be a touch sensor or the like and may indicate whether a user acts onto the actuation device for actuating the fastening element and/or the driving assembly.

A reading of the actuation sensing device indicative of an actuation of the actuation device may be used in the context of braking a movement of the pusher device. For example, a braking of the movement of the pusher device can be initiated if the fastening element is in an intermediate position and an actuation of the actuation element is detected.

A reading of the actuation sensing device indicative of an actuation of the actuation device may, alternatively or in addition, be also used in the context of terminating a braking of the movement of the pusher device.

For example, the control device may be configured to transfer the brake device from the braking state to the non-braking state if an output of the fastening element sensing device indicates that the fastening element is not in an intermediate position in between the non-actuated position and the fully actuated position and, in addition, an output of the actuation sensing device indicates that the dictation device is not actuated and/or an output of the clutch sensing device indicates that the driving assembly is in the clutched state. For example, if the fastening element sensing device indicates that the fastening element is not in an intermediate position and the actuation sensing device indicates that the actuation device is released, i.e., and not actuated (and/or the clutch sensing device indicates that the driving assembly is in the clutched state), this relates to a normal use situation in which the driving assembly is in the clutched state and hence controls movement of the pusher device.

Alternatively or in addition, the control device may be configured to transfer the brake device from the braking state to the non-braking state if an output of the fastening element sensing device indicates that the fastening element is not in an intermediate position in between the non-actuated position and the fully actuated position, and in addition an output of the actuation sensing device indicates that the actuation device is actuated and/or an output of the clutch sensing device indicates that the driving assembly is in the unclutched state. In this case the actuation device is fully actuated, such that no braking is necessary, but rather a movement of the pusher device shall be allowed in order to for example bring the pusher device into operative connection with the piston of a syringe for example in the context of an installation procedure.

In one embodiment, the infusion device comprises a movement sensing device for detecting a movement of the pusher device along the pushing direction, wherein the control device is configured to control the brake device to assume the braking state or the non-braking state based in addition on an output of the movement sensing device. By additionally using the movement sensing device, a braking can for example only be initiated if indeed the pusher device is moved while the fastening element sensing device indicates for example that the fastening element is in an intermediate position. Hence, a braking is only caused if necessary, hence if an actual risk exists that during installation the pusher device may be approached towards the piston while the fastening element is not in a position in which an operative connection between the pusher device and the piston may be established.

The object is also achieved by means of a method for controlling operation of an infusion device, the method comprising: providing a pusher device movable in a pushing direction for acting onto a piston of the syringe received in a receptacle of the infusion device; moving a fastening element arranged on the pusher device in between a non-actuated position and a fully actuated position with respect to the pusher device; braking, using a brake device, a movement of the pusher device; and controlling, using a control device, operation of the brake device. Herein, the method further comprises steps of sensing, using a fastening element sensing device, a position state of the fastening element with respect to the pusher device; and controlling, using the control device, the brake device, based on an output of the fastening element sensing device, to assume a braking state for braking a movement of the pusher device or a non-braking state for allowing a movement of the pusher device.

The advantages and advantageous embodiments described above for the infusion device equally apply also to the method, such that it shall be referred to the above in this respect.

The idea underlying the invention shall subsequently be described in more detail with respect to the embodiments shown in the figures. Herein:
- Fig. 1: shows a view of an embodiment of an infusion device in the shape of a syringe pump;
- Fig. 2: shows a schematic view of a drive mechanism of the infusion device;
- Fig. 3: shows a schematic view of a driving assembly of a drive element of the drive mechanism;
- Fig. 4: shows a view of an embodiment of a driving assembly comprising two clutch elements;
- Fig. 5: shows a sectional view of the driving assembly;
- Fig. 6: shows a view of an embodiment of a pusher device of an infusion device;
- Fig. 7A: shows a schematic view of a fastening element on a pusher device in a non-actuated position;
- Fig. 7B: shows a schematic view of a fastening element on a pusher device in an intermediate position in engagement with a small-diameter piston of a syringe;
- Fig. 7C: shows a schematic view of a fastening element on a pusher device in an intermediate position in engagement with a large-diameter piston of a syringe;
- Fig. 7D: shows a schematic view of a fastening element on a pusher device in a fully actuated position;
- Fig. 8: shows a schematic view of a control device of an infusion device for processing sensor outputs in the context of controlling operation of a brake device; and
- Fig. 9: shows a state diagram indicating different states of a brake device.

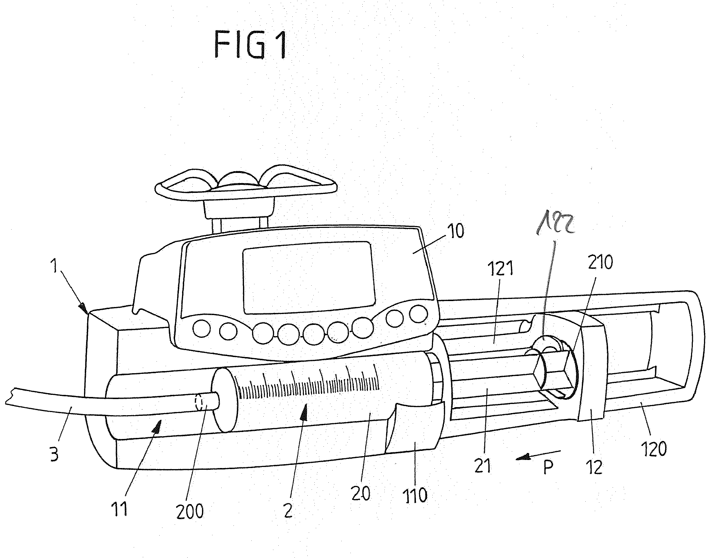

Fig. 1 shows an embodiment of an infusion device 1 in the shape of a syringe pump having a housing 10 and a receptacle 11 arranged on the housing 10 to receive a syringe 2 therein.

The syringe 2 comprises a cylindrical tube 20 which, when installing the syringe 2 on the infusion device 1, contains a medical liquid, for example a medication or a solution for the parenteral feeding, to be infused to a patient. The cylindrical tube 20 is connected, via a connector 200, to an infusion line 3 which may extend from the syringe 2 towards a patient for infusing the medical liquid to the patient.

For installing the syringe 2 on the receptacle 11 of the infusion device 1, the cylindrical tube 20 of the syringe 2 is placed in the receptacle 11 and is mechanically connected to the housing 10 by means of a fixation device 110. By means of the fixation device 110, for example configured by a releasable clamp element, the cylindrical tube 20 is secured within the receptacle 11 such that the cylindrical tube 20 is held in position on the receptacle 11.

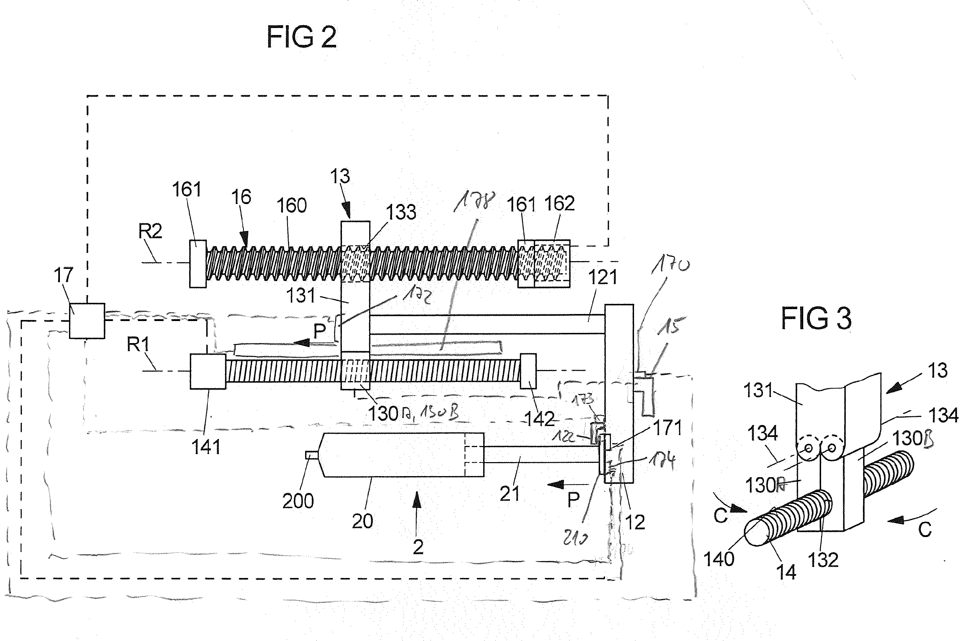

The syringe 2 comprises a piston 21 which, for delivering medical fluid contained in the cylindrical tube 20, can be pushed into the cylindrical tube 20 in a pushing direction P. For this, the infusion device 1 comprises a pusher device 12 movably arranged within a guide device 120 and connected to a drive mechanism (which subsequently shall be described with relation to Fig. 2 and 3) via a connecting rod 121.

For operating the infusion device 1, the syringe 2 is installed on the infusion device 1 and the pusher device 12 is (manually) moved towards a piston head 210 of the piston 21 until the pusher device 12 comes into abutment with the piston head 210. For performing an infusion process the pusher device 12 is then electromotively moved in the pushing direction P to move the piston 21 into the cylindrical tube 20 for delivering the medical fluid contained in the cylindrical tube 20 via the infusion line 3 towards the patient.

The pusher device 12 is driven by a drive mechanism, which, according to one embodiment, is schematically illustrated in Fig. 2. The drive mechanism comprises a driving assembly 13, which is connected to the pusher device 12 via the connecting rod 121 in a mechanically fixed manner such that by moving the driving assembly 13 the pusher device 12 is moved along the pushing direction P. The driving assembly 13 is movable within the housing 10 along the pushing direction P and, via clutch elements 130A, 130B, is (releasably) connected to a driving rod 14 having a screw thread 140.

A schematic, simplified drawing of a driving assembly 13 is shown in Fig. 3. The driving assembly 13 comprises two clutch elements 130A, 130B which each are pivotably connected, about an associated pivot axis 134, to a frame member 131 of the driving assembly 13. The clutch elements 130A, 130B each are shaped as a half nut and comprise a rod receptacle in the shape of screw thread 132 by which they may engage with the screw thread 140 of the driving rod 14.

In a clutched state, as illustrated in Fig. 3, the clutch elements 130A, 130B are pivoted towards each other in a clutching direction C such that they receive the driving rod 14 in between them and engage with the screw thread 140 of the driving rod 14. To release the engagement, the clutch elements 130A, 130B are pivoted opposite to the clutching direction C away from one another, such that they disengage from the screw thread 140 of the driving rod 14 and hence release the connection between the driving assembly 13 and the driving rod 14.

During regular infusion operation of the infusion device 1 the driving assembly 13 is in the clutched state in which the clutch elements 130A, 130B engage with the screw thread 140 of the driving rod 14. The driving rod 14, at one end, is connected to an electric drive motor 141 and at the other end is received in a bearing 142 such that, driven by the electric drive motor 141, the driving rod 14 can be rotated about an axis of rotation R1. By rotating the driving rod 14, the driving assembly 13 (due to the engagement of the clutch elements 130A, 130B with the screw thread 140 of the driving rod 14) is longitudinally moved along the driving rod 14, and by the movement of the driving assembly 13 the pusher device 12 pushes the piston 21 in the pushing direction P into the cylindrical tube 20 of the syringe 2.

The driving assembly 13 is operatively connected to a brake device 16 having a threaded spindle 160 which is rotatable, within bearings 161, about a rotational axis R2. The driving assembly 13 comprises an engagement opening 133 having a screw thread therein which engages with a screw thread of the threaded spindle 160. A longitudinal movement of the driving assembly 13 along the pushing direction P hence causes, due to the engagement, the threaded spindle 160 to be rotated about the rotational axis R2, which generally may be possible at low force if the screw thread of the threaded spindle 160 has a comparatively large pitch.

The threaded spindle 160, at one end, is associated with a brake 162 configured for example by an electromagnetic brake. If the brake 162 is actuated, it blocks a rotation of the threaded spindle 160 about its rotational axis R2. If the threaded spindle 160 is not able to rotate, the driving assembly 13 cannot move along the threaded spindle 160 such that the driving assembly 13 is held in position and hence is braked by the brake device 16. If the brake 162 in contrast is deactivated, the threaded spindle 160 is allowed to rotate, such that the driving assembly 13 is not braked and may be moved longitudinally along the threaded spindle 160.

The operation of the infusion device 1 is controlled by means of a control device 17. In particular, the control device 17 acts onto the electric drive device 141 to rotate the driving rod 14, and the control device 17 acts onto the brake 162 to switch the brake device 16 between its activated and its deactivated state.

The driving assembly 13 is actuatable by means of an actuation device 15, for example in the shape of a lever arranged on the pusher device 12. Herein, the lever may be manually pressed to unclutch the clutch elements 130A, 130B from the driving rod 14, and may be released in order to revert the driving assembly 13 to its clutched state.

The drive mechanism as schematically illustrated in Fig. 2 and 3 may, in one embodiment, be implemented by a mechanism as it is described for example in US 2012/015170 A1, which shall be incorporated by reference herein.

For installing a syringe 2 on the infusion device 1 the pusher device 12 is manually moved such that it comes into contact with the piston 21. For this, the driving assembly 13 is actuated by means of the actuation device 15 to move the clutch elements 130A, 130B to their unclutched state, such that the driving assembly 13 can freely be moved along the driving rod 14. Once the pusher device 12 has come into contact with the piston head 210 of the piston 21, the clutch elements 130A, 130B are brought into engagement with the driving rod 14 by releasing the lever of the actuation device 15.

In addition, during operation of the infusion device it is conceivable that a user may want to perform a manual bolus by manually pushing the pusher device 12 in the pushing direction P to push the piston 21 into the cylindrical tube 20. For this, the clutch elements 130A, 130B are unclutched from the driving rod 14 such that the pusher device 12 is manually movable, and, after the manual bolus has been performed, are reverted to their clutched state such that the regular infusion operation may resume.

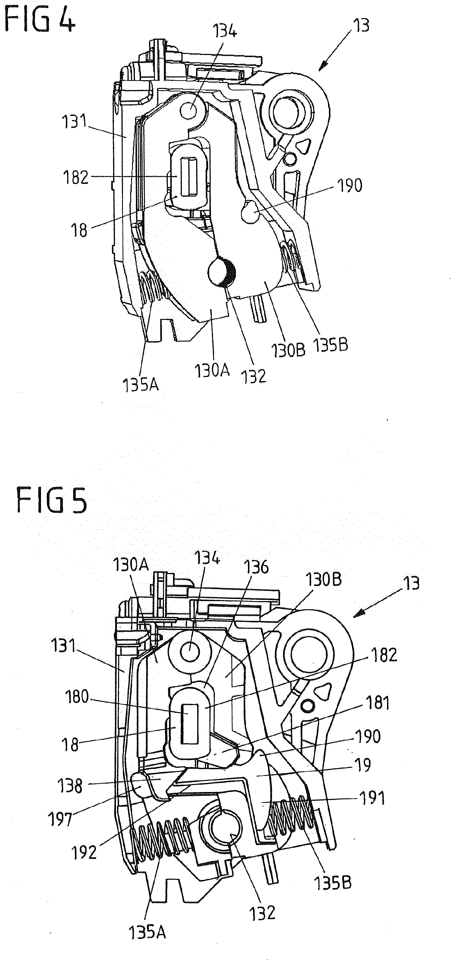

Referring now to Figs. 4 and 5, in an embodiment the driving assembly 13 may comprise a frame member 131 to which two clutch elements 130A, 130B are pivotably connected via a common pivot axis 134. Each clutch element 130A, 130B comprises a rod receptacle 132 in the shape of a screw thread such that, in a clutched state of the driving assembly 13, the driving rod 14 is received in between the clutch elements 130A, 130B and the rod receptacle 132 of each clutch element 130A, 130B engages with the screw thread 140 of the driving rod 14. As described above, a rotational movement of the driving rod 14 hence causes a longitudinal movement of the frame member 131 along the driving rod 14, the connecting rod 121 being fixedly connected to the frame member 131 such that the pusher device 12 is moved along with the frame member 131.

Each clutch element 130A, 130B is elastically tensioned with respect to the frame member 131 by means of a spring element 135A, 135B in the shape of a push spring. The tensioning herein acts towards the clutched state such that the clutch elements 130A, 130B are pushed towards each other for engaging with the driving rod 14 placed in between the clutch elements 130A, 130B.

In the embodiment of Figs. 4 and 5, a lock element 19 is pivotably arranged, via a pivot axis 190, on a first clutch element 130B of the two clutch elements 130A, 130B. The lock element 19 comprises a lock arm 192 reaching from the pivot axis 190 towards the other, second clutch element 130A and having a locking contour 197 engaging, in the clutched state of the driving assembly 13, with a locking protrusion 138 of the other, second clutch element 130 A, as shown in Fig. 5.

Via the lock element 19 the clutch elements 130A, 130B hence are locked with respect to each other in the clutched state of the driving assembly 13, such that forces acting onto the driving assembly 13 - due to for example a pressure rise in the syringe 2 or in the infusion line 3 connected to the syringe 2 - may not lead to an opening of the driving assembly 13. The lock element 19 comprises, at approximately a perpendicular angle with respect to the lock arm 192, a tensioning arm 191 which is in abutment with the spring element 135B associated with the first clutch element 130B. By acting onto the tensioning arm 191 the spring element 135B indirectly acts onto the first clutch element 130B towards the clutched state, wherein the spring element 135B tensions the lock element 19 towards a locking position in which the lock element 19 with the lock arm 192 is in locking engagement with the locking protrusion 138 of the other, second clutch element 130A. When the driving assembly 13 is in the clutched state, thus, the lock element 19 automatically is moved to its locked position such that the clutch elements 130A, 130B are locked with respect to each other and cannot be moved apart from one another without unlocking the lock element 19.

The unclutching of the driving assembly 13 takes place via a cam element 18 having a body portion 182 received in an inner space 136 in between the clutch elements 130A, 130B. The cam element 18 is in connection with a shaft 180 being in operative connection with the actuation device 15 and being pivoted upon actuation of the actuation device 15. The cam element 18 serves to push apart the clutch elements 130A, 130B in a releasing direction R. By moving the body portion 182, the clutch elements 130A, 130B are pushed apart against the tensioning forces of the spring elements 135A, 135B, such that the engagement of the rod receptacles 132 of the clutch elements 130A, 130B with the driving rod 14 may be released. The cam element 18, when actuated to unclutch the driving assembly 13, also acts onto the lock element 19. For this, the cam element 18 comprises a lever arm 181 protruding from the body portion 182 and being in abutment with the lock arm 192 of the lock element 19. When the cam element 18 is pivoted for unclutching the driving assembly 13, the lever arm 181 acts onto the lock arm 192 of the lock element 19 and pushes the lock arm 192 downwards such that the engagement of the locking contour 197 with the locking protrusion 138 is released.

Referring now again to Fig. 2, the infusion device 1 comprises a fastening element 122 movably placed on the pusher device 12. The fastening element 122 serves to establish an operative connection in between the pusher device 12 and the piston 21 of a syringe 2 in order to fasten the piston head 210 on the pusher device 12. By means of the fastening element 122, also denoted as anti-siphon arm, the piston 21 is held in abutment with the pusher device 12, allowing in particular also to cause a movement of the piston 21 opposite to the pushing direction P by movement of the pusher device 12. In addition, by means of the fastening element 122 a movement of the piston 21 independent of the pusher device 12, for example in case of an underpressure within the cylindrical tube 20, is avoided by fixing the piston 21 with respect to the pusher device 12.

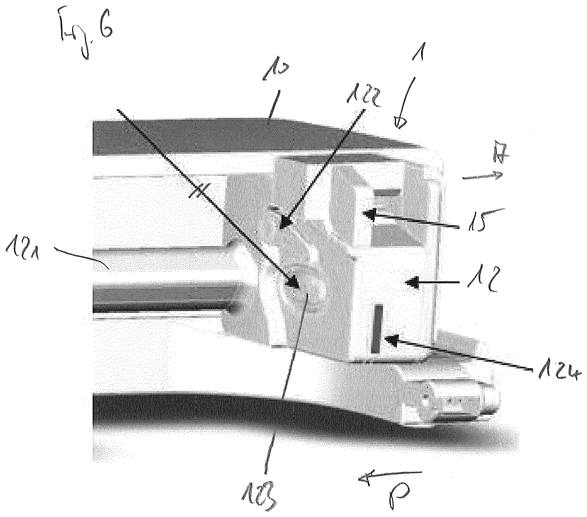

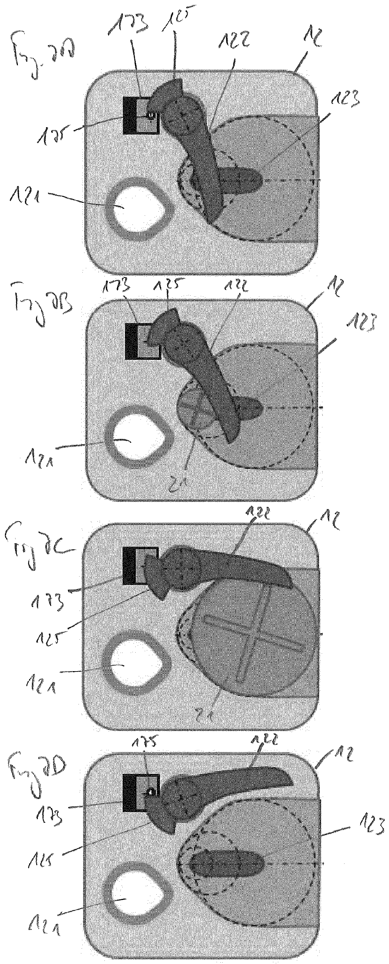

Referring now to Fig. 6 und Figs. 7A to 7D, the fastening element 122 may have the shape of a lever which is pivotably arranged on the pusher device 12 and is movable on the pusher device 12 in between a non-actuated position (Fig. 7A) and a fully actuated position (Fig. 7D). The fastening element 122 herein is actuatable together with the driving assembly 13 by means of the actuation device 15, such that by actuating the actuation device 15 the driving assembly 13 can be brought into its unclutched state and at the same time the fastening element 122 is mechanically moved towards its fully actuated position.

The fastening element 122 herein is biased towards its non-actuated position (Fig. 7A) for example by means of an elastic spring, such that the fastening element 122 assumes the non-actuated position in case the actuation device 15 is not actuated.

If a piston 21 is in operative connection with the pusher device 12, the fastening element 122 engages with the piston 21 and fixes the piston 21 on the pusher device 12. In this case, as visible from Fig. 7B and 7C, the fastening element 122 assumes an intermediate position in between the non-actuated position (Fig. 7A) and the fully actuated position (Fig. 7B) by resting on the piston 21.

In the embodiment of Fig. 6, the actuation device 15 has the shape of a lever which is slidably arranged on the pusher device 12 and can be moved in an actuation direction A for actuating the actuation device 15 and in this way to move the fastening element 122 towards the fully actuated position (Fig. 7A) and to transfer the driving assembly 13 to the unclutched state.

Referring now again to Fig. 2, the infusion device 1 comprises a multiplicity of sensing devices 170-174, 178.

An actuation sensing device 170 serves to detect a state of the actuation device 15. The actuation sensing device 170 may for example be a touch sensor, a micro-switch or a photocell for detecting whether the actuation device 15 is actuated from a rest position.

A force sensor 171 serves to measure a force in between the pusher device 12 and the piston 21 of a syringe 2 installed on the infusion device 1 during a regular infusion operation.

A clutch sensing device 172 serves to sense a state of the driving assembly 13, namely whether the driving assembly 13 is in the unclutched state or the clutched state. The clutch sensing device 172 may for example be a photocell or a micro-switch.

A fastening element sensing device 173 serves to detect a position state of the fastening element 122 and may have for example the shape of a photocell or a micro-switch for detecting whether the fastening element 122 assumes a specific, discrete position.

In the embodiment of Figs. 7A to 7D, the fastening element sensing device 173 comprises a photocell 175 which interacts with an end section 125 of the fastening element 122 such that the fastening element sensing device 172 may detect (by a binary true-false reading) whether the fastening element 122 is in one of its end positions (Figs. 7A, 7D) or in an intermediate position (Figs. 7B, 7C).

A presence sensing device 174 may serve to detect whether a piston 21 by means of its piston head 210 rests on a shim element 123 (see Fig. 6) of the pusher device 12. The presence sensing device 174 may for example have the shape of a photocell or a micro-switch.

A movement sensing device 178, for example in the shape of a linear potentiometer, may serve to detect a movement of the driving assembly 13 along the pushing direction P, for example by continuously detecting the position of the driving assembly 13 along the pushing direction P.

Outputs of all sensing devices 170-174, 178 are provided to the control device 17, which serves to process the sensor outputs and to control operation in particular of the drive motor 141 and the brake device 16.

When a syringe 2 is to be installed on the infusion device 1 and hence shall be brought into operative connection with the pusher device 12, the syringe 2 is placed on the receptacle 11 and the actuation device 15 is actuated to transfer the driving assembly 13 to the unclutched state, such that the pusher device 12 may be approached, in the pushing direction P, towards the piston head 210 of the piston 21 for establishing an operative connection in between the pusher device 12 and the piston 21. By actuating the actuation device 15, also the fastening element 122 is moved from its rest position, namely the non-actuated state according to Fig. 7A.

Whereas the actuation of the driving assembly 13 and the fastening element 122 is caused by the same actuation of the actuation device 15, the points in time at which the driving assembly 13 reaches its unclutched state and the fastening element 122 reaches its fully actuated position (Fig. 7D) may differ, the driving assembly 13 reaching the unclutched state generally before the fastening element 122 actually is in the fully actuated position.

If a user, however, would start moving the pusher device 12 before the fastening element 122 has reached its fully actuated position, it may come about that the fastening element 122 collides with the piston head 210 when approaching the pusher device 12 towards the piston head 210. Conventionally, once it is detected - for example by means of the presence sensing device 174 - that an operative connection in between the pusher device 12 and the piston 21 has been established, the brake device 16 is actuated such that a further movement of the pusher device 12 is prohibited. However, if the fastening element 122 during the approaching movement of the pusher device 12 collides with the piston head 210, the presence sensing device 174 may never detect a presence of the piston 21, such that no braking is initiated and, potentially, a bolus may be caused by acting onto the piston 21 during the installation procedure.

For this reason, it herein is proposed to process outputs of the sensing devices 170-174, 178 in order to cause a braking of the movement of the pusher device 12 in case the fastening element 122 is not correctly actuated, for example during an installation procedure or during an uninstallation procedure.

In one embodiment, if (e.g., during an installation procedure) it is for example detected that the driving assembly 13 is in the unclutched state (by means of the clutch sensing device 172), if in addition the fastening element sensing device 173 indicates an intermediate position of the fastening element 122, and if the presence sensing device 174 indicates no presence of a piston 21 on the pusher device 12, the brake device 16 may be switched, by means of the control device 17, to a braking state for braking a movement of the pusher device 12, such that a movement of the pusher device 12 is prohibited.

In another embodiment, a breaking may be caused only based on an output of the fastening element sensing device 173 and the presence sensing device 174, but not taking into account an output of the clutch sensing device 172. Hence, if (e.g., during installation procedure) it is for example detected that the fastening element sensing device 173 indicates an intermediate position of the fastening element 122, and if the presence sensing device 174 indicates no presence of a piston 21 on the pusher device 12, the brake device 16 may be switched, by means of the control device 17, to the braking state for braking a movement of the pusher device 12, such that a movement of the pusher device 12 is prohibited. This may allow for a faster braking of the pusher device 12, hence preventing a movement of the pusher device 12 early on.

Hence, if during an installation procedure it is detected that the fastening element 122 has not been fully and correctly brought into the fully actuated state (Fig. 7D), it is prevented that the pusher device 12 may be approached towards the piston 21 of a syringe 2 received on the infusion device 1. Hence, a collision of the fastening element 122 with the piston head 210 during installation is prevented, thus avoiding a risk for an undesired bolus.

Also, if during an uninstallation procedure the fastening element 122 has not been fully and correctly brought into the fully actuated state for releasing the operative connection in between the pusher device 12 and the piston 21, a movement of the pusher device 12 is prevented, such that the piston 21 may not be carried along when for example moving the pusher device 12 opposite to the pushing direction P.

In addition, a sensor output of the movement sensor 178 may be taken into account. For example, a braking may only be initiated if it is found that the driving assembly 13 and hence the pusher device 12 is actually moved, which can be detected by means of the movement sensing device 178.

The braking generally is released if it is found that the fastening element 122 has correctly been brought into its fully actuated state (Fig. 7D).

Specifically, in one embodiment, the braking may be stopped if it is detected that the actuation device 15 is released (which is detected by the actuation sensing device 170) and if in addition the fastening element 122 is not in an intermediate position, but in one of its end positions (which is detected by means of the fastening element sensing device 173).

In addition, the braking may be stopped if it is detected that the actuation device 15 is actuated (which is detected by the actuation sensing device 170), if in addition the fastening element 122 is not in an intermediate position, but in one of its end positions (which is detected by means of the fastening element sensing device 173), and if the driving assembly 13 is in the unclutched state (which is detected by means of the clutch sensing device 172).

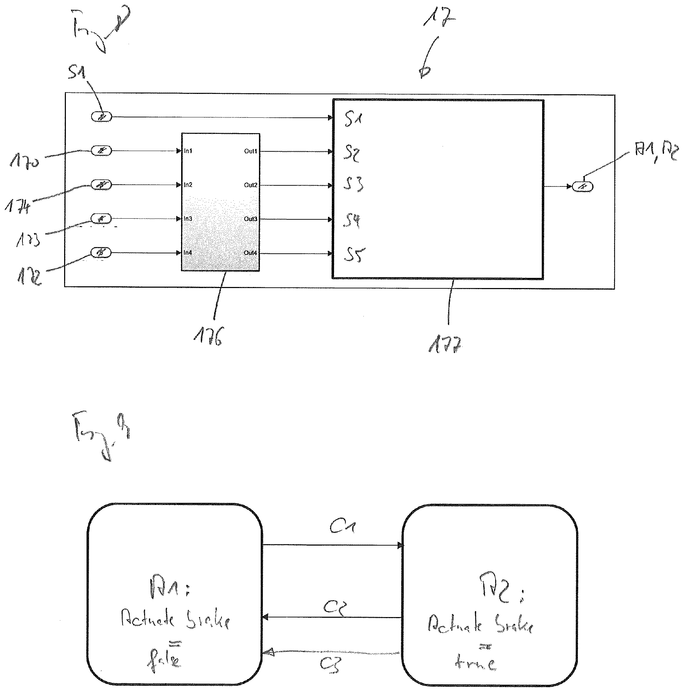

Referring now to Fig. 8, outputs of the sensing devices 170-174, 178 are processed by the control device 17. Sensor outputs herein may be fed to a pre-processing module 176, which may derive, in a pre-processing treatment, from the sensor outputs specific state information S2-S5 which may then be fed to a processing module 177. The pre-processing treatment to derive state information S2-S5 may allow for a fast processing of information in order to be able to switch the brake device 16 between its different states with a fast reaction time to the sensor outputs.

In particular, the preprocessing module 176 may derive, from the outputs of the actuation sensing device 170, from the clutch sensing device 172, from the fastening element sensing device 173 and from the presence sensing device 174 state information S2-S5 indicating whether the actuation device 15 is pressed (state information S2), a piston 21 is present on the pushing device 12 (state information S3), the fastening element 122 is in the intermediate position (state information S4), and the driving assembly 13 is in the clutched state or unclutched state (state information S5).

In addition, a state information S1 relating to whether a piston 21 is installed on the pusher device 12 may be fed to the processing module 177, which processes the different state information S1-S5 in order to control the brake device 16 to assume a braking state or a non-braking state.

Referring now to Fig. 9 illustrating a state diagram for state changes of the brake device 16, in a state A1 the brake device 16 is in the non-braking state in which a movement of the pusher device 12 is possible. In a state A2, in contrast, the brake device 16 is in the braking state in which the pusher device 12 is held in place and hence is prevented from moving along the pushing direction P.

If the brake device 16 is in the non-braking state A1, the brake device 16 may transition to the braking state A2 in case it is detected that no syringe piston 21 is installed on the pusher device 12 (state information S1), it is further detected that the fastening element 122 is in an intermediate position, the driving assembly 13 is in the unclutched state and no piston 21 is detected as present by the presence sensing device 174 (condition C1). If the brake device is in the braking state A2, the brake device 16 may transition to the braking state A2 in case it is detected that the actuation device 15 is actuated, the driving assembly 13 is in the unclutched state, and the fastening element 122 is not in an intermediate position (condition C2).

In addition, the brake device 16 may transition to the braking state A2 in case it is detected that the actuation device 15 is not actuated, and the fastening element 122 is not in an intermediate position (condition C3).

The idea underlying the invention is not limited to the embodiments described above, but may be implemented in an entirely different fashion in entirely different embodiments.

An infusion device of the kind described herein may serve different purposes and may in particular be used to deliver a medical fluid such as a medication or a nutritional fluid, for example for the enteral or parenteral feeding, towards a patient.

### List of Reference Numerals

- 1: Infusion device
- 10: Housing
- 11: Receptacle
- 110: Fixation device
- 12: Pusher device
- 120: Guide device
- 121: Connecting rod
- 122: Fastening element (anti-siphon arm)
- 123: Shim element
- 124: Indicator device
- 125: End section
- 13: Driving assembly
- 130A, 130B: Clutch element
- 131: Frame member
- 132: Rod receptacle
- 133: Engagement opening
- 134: Pivot axis
- 135A, 135B: Spring element
- 136: Inner space
- 138: Locking protrusion
- 14: Driving rod (spindle)
- 140: Screw thread
- 141: Drive motor
- 142: Bearing
- 15: Actuation device (handle)
- 16: Brake device
- 160: Threaded spindle
- 161: Bearing
- 162: Brake
- 17: Control device
- 170-174, 178: Sensing device
- 175: Photo cell
- 176: Pre-processing module
- 177: Processing module
- 18: Cam element
- 180: Shaft
- 181: Lever arm
- 182: Body portion
- 19: Lock element
- 190: Pivot axis
- 191: Tensioning arm
- 192: Lock arm
- 197: Locking contour
- 2: Syringe
- 20: Cylinder tube
- 200: Connector
- 21: Piston
- 210: Piston head
- 3: Infusion line
- A: Actuation direction
- A1, A2: Actuation state
- C: Clutching direction
- C1-C3: Condition
- P: Pushing direction
- R1, R2: Axis of rotation
- S1-S5: State

## Claims

1. Infusion device (1) for administering a medical fluid to a patient, comprising:
a receptacle (11) for receiving a syringe (2),
a pusher device (12) movable in a pushing direction (P) for acting onto a piston (21) of a syringe (2) received in the receptacle (11),
a fastening element (122) arranged on the pusher device (12) and movable in between a non-actuated position and a fully actuated position with respect to the pusher device (12),
a brake device (16) for braking a movement of the pusher device (12), and
a control device (17) for controlling operation of the brake device (16),
**characterized by**
a fastening element sensing device (173) for sensing a position state of the fastening element (122) with respect to the pusher device (12), wherein the control device (17) is configured to control the brake device (16), based on an output of the fastening element sensing device (173), to assume a braking state for braking a movement of the pusher device (12) or a non-braking state for allowing a movement of the pusher device (12).

2. Infusion device (1) according to claim 1, **characterized in that** the control device (17) is configured to control the brake device (16) based on an output of the fastening element sensing device (173) and an output of at least one further sensing device (170-172, 174).

3. Infusion device (1) according to claim 1 or 2, **characterized in that** the control device (17) is configured to control the brake device (16) to assume the braking state if an output of the fastening element sensing device (173) indicates that the fastening element (122) is in an intermediate position in between the non-actuated position and the fully actuated position and if in addition an output of at least one further sensing device (170-172, 174) indicates that the pusher device (12) is not in operative connection with a piston (21) of a syringe (2).

4. Infusion device (1) according to one of claims 1 to 3, **characterized by** a presence sensing device (174) for sensing a presence of a piston (21) of a syringe (2) on the pusher device (12), wherein the control device (17) is configured to control the brake device (16) to assume the braking state or the non-braking state based in addition on an output of the presence sensing device (174).

5. Infusion device (1) according to one of the preceding claims, **characterized by** a driving rod (14), an electric drive device (141) for driving the driving rod (14), and a driving assembly (13) comprising at least one clutch element (130A, 130B), the at least one clutch element (130A, 130B) being configured to operatively connect, in a clutched state, the driving rod (14) to the pusher device (12) for driving the pusher device (12) by the electric drive device (141), the driving assembly (13) being actuatable to assume an unclutched state in which the operative connection between the driving rod (14) and the pusher device (12) is released.

6. Infusion device (1) according to claim 5, **characterized by** a clutch sensing device (172) for sensing a state of the driving assembly (13), wherein the control device (17) is configured to control the brake device (16) to assume the braking state or the non-braking state based in addition on an output of the clutch sensing device (172).

7. Infusion device (1) according to claim 5 or 6, **characterized by** an actuation device (15) for actuating at least one of the fastening element (122) to move the fastening element (122) with respect to the pusher device (12) and the driving assembly (13) to assume the unclutched state.

8. Infusion device (1) according to claim 7, **characterized in that** the actuation device (15) is movably arranged on the pusher device (12).

9. Infusion device (1) according to claim 7 or 8, **characterized by** an actuation sensing device (170) for sensing a state of the actuation device (15), wherein the control device (17) is configured to control the brake device (16) to assume the braking state or the non-braking state based in addition on an output of the actuation sensing device (170).

10. Infusion device (1) according to claim 9, **characterized in that** the control device (17) is configured to transfer the brake device (16) from the braking state to the non-braking state if
an output of the fastening element sensing device (173) indicates that the fastening element (122) is not in an intermediate position in between the non-actuated position and the fully actuated position and
in addition at least one of an output of the actuation sensing device (170) indicates that the actuation device (15) is not actuated and an output of the clutch sensing device (172) indicates that the driving assembly (13) is in the clutched state.

11. Infusion device (1) according to claim 9 or 10, **characterized in that** the control device (17) is configured to transfer the brake device (16) from the braking state to the non-braking state if
an output of the fastening element sensing device (173) indicates that the fastening element (122) is not in an intermediate position in between the non-actuated position and the fully actuated position and
in addition at least one of an output of the actuation sensing device (170) indicates that the actuation device (15) is actuated and an output of the clutch sensing device (172) indicates that the driving assembly (13) is in the unclutched state.

12. Infusion device (1) according to one of the preceding claims, **characterized by** a movement sensing device (178) for detecting a movement of the pusher device (12) along the pushing direction (P), wherein the control device (17) is configured to control the brake device (16) to assume the braking state or the non-braking state based in addition on an output of the movement sensing device (178).

13. Method for controlling operation of an infusion device (1), comprising:
providing a pusher device (12) movable in a pushing direction (P) for acting onto a piston (21) of a syringe (2) received in a receptacle (11) of the infusion device (1),
moving a fastening element (122) arranged on the pusher device (12) in between a non-actuated position and a fully actuated position with respect to the pusher device (12),
braking, using a brake device (16), a movement of the pusher device (12), and
controlling, using a control device (17), operation of the brake device (16),
**characterized by**
sensing, using a fastening element sensing device (173), a position state of the fastening element (122) with respect to the pusher device (12), and controlling, using the control device (17), the brake device (16), based on an output of the fastening element sensing device (173), to assume a braking state for braking a movement of the pusher device (12) or a non-braking state for allowing a movement of the pusher device (12).
